(19) Europäisches Patentamt
European Patent Office
Office européen des brevets

(11) Publication number: 0 115 251 B1

# (12) EUROPEAN PATENT SPECIFICATION

(45) Date of publication of patent specification: 14.05.86

(51) Int. Cl.⁴: C 07 C 149/24, C 11 D 1/10, C 11 D 1/18, B 01 F 17/00

(21) Application number: 83810609.4

(22) Date of filing: 22.12.83

## (54) Perfluoroalkyl amphoteric compounds.

(30) Priority: 29.12.82 US 454211

(43) Date of publication of application:
08.08.84 Bulletin 84/32

(45) Publication of the grant of the patent:
14.05.86 Bulletin 86/20

(84) Designated Contracting States:
BE CH DE FR GB IT LI

(56) References cited:
DE-A-2 018 461
US-A-3 948 887
US-A-4 126 633

(73) Proprietor: CIBA-GEIGY AG
Klybeckstrasse 141
CH-4002 Basel (CH)

(72) Inventor: Falk, Robert A.
35 Glenside Drive
New City New York 10956 (US)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 115 251 B1

**Description**

The present invention relates to perfluoroalkyl alkylene and thioalkylene containing amphoteric compounds, which are useful as surface active agents.

Amphoteric surfactants are known in the art. US 3.480.666, for example, describes amphoteric sulfonate surfactants containing hydroxyl groups and which are derived from non-fluorochemical epoxides. Further fluorinated amphoteric surfactants which are derived from unsaturated glycidyl ethers, are disclosed in US 4.038.195.

It is one object of the present invention to provide new perfluoroalkyl amphoteric compounds of the formula

$$R_f{-}R_1{-}SCH_2CR_2(OH)CHR_3\overset{\overset{\displaystyle R_5}{|}}{\underset{\underset{\displaystyle R_4}{|}}{N^{\oplus}}}{-}Z{-}Y^{\ominus} \qquad (1)$$

wherein

$R_f$ is perfluoroalkyl of 6 to 12 carbon atoms or said perfluoroalkyl substituted by perfluoroalkoxy of 2 to 6 atoms.

$R_1$ is alkylene of 2 to 8 carbon atoms, alkylenethioalkylene of 2 to 8 carbon atoms, alkyleneoxyalkylene of 2 to 8 carbon atoms or alkyleneiminoalkylene of 2 to 8 carbon atoms where the nitrogen atom contains hydrogen or lower alkyl as a third substituent,

$R_2$ and $R_3$ are independently hydrogen or lower alkyl which is unsubstituted or substituted by lower alkoxy or halogen, cycloalkyl of 5 to 6 carbon atoms or alkenyl of up to 6 carbon atoms or when taken together with or without a sulfur or oxygen atom, $R_2$ and $R_3$ contain up to 6 alkylene carbon atoms,

$R_4$ and $R_5$ are independently hydrogen or alkyl of 1 to 12 carbon atoms or phenyl which are unsubstituted or substituted by carboxy, sulfo, sulfato, phosphoro or phosphono and

Z is alkylene of 1 to 12 carbon atoms and Y is carboxy, sulfo, sulfato, phosphoro, phosphono or thiosulfato,

or the acid adduct, alkaline salt, or N-lower alkyl or N-benzyl quaternary ammonium salts of the compounds of the formula (1).

Another object of the present invention is a process for the preparation of the compounds of the formula (1).

In the compounds of the formula (1), $R_f$ is a perfluoroalkyl radical containing preferably 6 to 12 carbon atoms and is thus represented by perfluorinated hexyl, heptyl, octyl, nonyl, decyl, undecyl and dodecyl and isomers thereof. The perfluoroalkyl radicals $R_f$ are unsubstituted or substituted by perfluoroalkoxy groups of preferably 2 to 6 carbon atoms such as perfluorinated ethoxy, propoxy, butoxy, pentoxy and hexoxy and isomers thereof.

Most preferably, however, $R_f$ denotes an unsubstituted perfluoroalkyl radical.

The linking group $R_1$ is an alkylene moiety such as alkylene containing 2 to 8 carbon atoms, where ethylene, propylene and butylene are particularly preferred, further alkylenethioalkylene, alkyleneoxyalkylene and alkyleneiminoalkylene, preferably of the fomula $-(CH_2)_{1-7}-X-(CH_2)_{1-7}-$, wherein X is $-S-$, $-O-$ or $-N(R)-$, R being hydrogen or lower alkyl.

It should be noted, that throughout the present specification "lower" in lower alkyl or lower alkoxy denotes those radicals which have 1 to 6, preferably 1 to 4 carbon atoms. More preferably, lower alkyl includes methyl and ethyl.

$R_2$ is hydrogen or lower alkyl such as methyl, ethyl, propyl or hexyl which is unsubstituted or substituted by lower alkoxy such as methoxy, ethoxy or butoxy or halogen such as chlorine or bromine. Further, $R_2$ denotes cycloalkyl of 5 to 6 carbon atoms, preferably cyclopentane or cyclohexane, and alkenyl of up to 6 carbon atoms such as ethenyl, propenyl, butenyl or hexenyl.

$R_3$ has independently from $R_2$ the same meanings as $R_2$.

$R_2$ and $R_3$ can form together an alkylene chain of up to 6 carbon atoms said chain being optionally interrupted by sulfur, nitrogen or oxygen to yield for example a morpholino, piperidino or thiomorpholino ring.

$R_4$ is hydrogen or alkyl having preferably 1 to 12 carbon atoms. Suitable alkyl radicals are thus for example methyl, ethyl, propyl, butyl, hexyl, oxtyl, decyl and dodecyl and isomers thereof. Preferred are the alkyl radicals of 1 to 6 carbon atoms and more preferably $R_4$ is methyl. Further, $R_4$ denotes phenyl. Said alkyl radicals and the phenyl radical can be substituted by carboxy ($-COOH$, $-COO^{\ominus}M^{\oplus}$, where $M^{\oplus}$ is a metal, preferably alkali metal ion, or an ammonium ion), sulfo ($-SO_3H$, $-SO^{\ominus}{}_3M^{\oplus}$ wherein $M^{\oplus}$ is as defined above), sulfato ($-OSO_3H$, $-OSO_3{}^{\ominus}M^{\oplus}$, $M^{\oplus}$ being as defined above), phosphoro ($-PO_4H_2$, $-PO_4H^{\ominus}M^{\oplus}$, $-PO_4{}^{2\ominus}M^{\oplus}{}_2$, $M^{\oplus}$ being as defined above) or phosphono ($-PO_3H_2$, $-PO_3H^{\ominus}M^{\oplus}$, $-PO_3{}^{2\ominus}M^{\oplus}{}_2$, $M^{\oplus}$ being as defined above). Preferred substituents are carboxy and sulfo.

$R_5$ has independently from $R_4$ the same meaning as $R_4$.

Z is alkylene having preferably 1 to 12 carbon atoms. Suitable radicals are derivable from the corresponding alkyl radicals listed in the definition of $R_4$. Preferably, Z is alkylene having 1 to 3 carbon atoms.

Y is an acid radical and denotes for example carboxy, sulfo, sulfato, phosphoro, phosphono (formulae of these radicals are given in the definition of $R_4$) or thiosulfato ($—S_2O_3H$, $—S_2^{\ominus}O_3M^{\oplus}$, wherein $M^{\oplus}$ is as defined above) or, preferably, carboxy or sulfo.

The compounds of the formula (1) can also be present in the form of an alkaline salt. Alkaline salts include those of the alkali metals, preferably sodium and potassium, and alkaline earth metals such as magnesium, calcium and barium.

The compounds of the formula (1) can further be present in the form of a quaternary ammonium salt, preferably a quaternary lower alkyl, lower alkanol or benzyl ammonium salt.

The acid adduct of the compounds of the formula (1) include adducts with conventional inorganic acids, especially the adducts of hydrochloric, hydrobromic, sulfuric, nitric and carbonic acids, and the conventional organic acids, including the lower alkanoic, lower alkylsulfonic, arylcarboxylic, preferably benzoic, and arylsulfonic, preferably tolylsulfonic, acids.

Preferred compounds of the formula (1) are those wherein $R_f$ is perfluoroalkoxy of 6 to 12 carbon atoms or said perfluoroalkyl substituted by perfluoroalkoxy of 2 to 6 atoms.

$R_1$ is alkylene of 2 to 8 carbon atoms, alkylenethioalkylene of 2 to 8 carbon atoms, alkyleneoxyalkylene of 2 to 8 carbon atoms or alkyleneiminoalkylene of 2 to 8 carbon atoms where the nitrogen atom contains hydrogen or alkyl 1 to 6 carbon atoms as a third substituent,

$R_2$ and $R_3$ are hydrogen,

$R_4$ and $R_5$ are hydrogen, methyl or alkyl of 1 to 6 carbon atoms substituted by carboxy,

Z is alkylene of 1 to 3 carbon atoms and

Y is carboxy or sulfo,

or the acid adduct, alkaline salt or N-lower alkyl quaternary ammonium salt thereof.

Particularly preferred are those compounds where $R_f$ is perfluoroalkyl of 6 to 12 carbon atoms, $R_1$ is alkylene of 2 to 4 carbon atoms, $R_2$ and $R_3$ are hydrogen, $R_4$ and $R_5$ are hydrogen, methyl or lower alkyl substituted by carboxy, Z is alkylene of 1 to 3 carbon atoms and

Y is carboxy or sulfo,

or the acid aduct, alkaline salt or N-lower alkyl quaternary ammonium salt thereof.

The novel $R_f$-amphoterics are obtained by the addition of $R_f$-epoxides of the formula

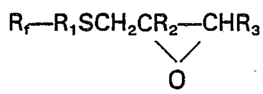

(2)

to amino acids of the formula

$$NR_4R_5ZY \quad (3)$$

wherein $R_f$, $R_1$, $R_2$, $R_3$, $R_4$, $R_5$, Z and Y are defined above.

The amino acids of use in this invention include both naturally occuring and synthetic amino acids containing not only the common carboxy and sulfo substituents but also sulfate, phosphoro and phosphono groups. These include for example glycine, alanine, valine, leucine, isoleucine, phenylalanine, proline, methionine, serine, threonine, cysterine, tyrosine, tryptophan, aminoadipic acid, alanine, methylalenine, amino butyric acid, ethylglycine, norvaline, aspartic acid, sarcosine, iminodiacetic acid, aminobezoic acid, 2-aminoethyl hydrogen sulfate, 2-aminoethylthiosulfuric acid, 2-aminoethyl hydrogen phosphate, taurine, methyl taurine.

The addition of epoxide to amino acid is a base catalyzed reaction which occurs most readily in a single phase. This is generally provided in an aqueous solvent mixture by choice of a suitable water miscible cosolvent such as methanol, isopropanol, butoxyethanol and the like. The reaction is generally conducted at temperatures from 25° to 200°C, but preferably from 50° to 130°C.

The reaction can be run uncatalyzed if the reactant is sufficiently basic or at an alkaline pH-value provided by alkali hydroxide, ion-exchange catalyst or a non-reactive base, e.g. triisopropylamine.

The products are generally useful without isolation but can be isolated depending on the pH-value, in acid foam, as isoelectronic neutral salts or as alkali metal or ammonium salts. The fully quaternized derivatives containing N-lower alkyl or N-benzyl quaternary functions are generally isolated as neutral zwitterionic compounds or acid adducts, e.g. hydrochlorides.

The subject amphoteric compounds are very stable to hydrolysis and find numerous uses as surfactants and wetting agents. Since they are derived from readily available amino acids and certain readily available fluorinated epoxides they comprise particularly useful compositions.

The following examples serve to illustrate the present invention.

Example 1

$C_8F_{17}CH_2CH_2SCH_2CHOHCH_2{}^{\oplus}NH(CH_3)CH_2CO^{\ominus}{}_2$

A 50 ml flask was charged with 3-(1,1,2,2-tetrahydroperfluorodecanethio)-1,2-epoxypropane (5.4 g; 10 mmoles), sarcosine (1.0 g; 10.5 mmoles), 50% aqueous sodium hydroxide (0.9 g; 11 mmoles), isopropanol (18 g) and sufficient water (5g) to attain a clear solution. The solution was heated to 77°C for 5 hours. After cooling, the solvent was removed by evaporation and the recovered solids were redissolved in water. The insolubles were filtered off, the filtrate dialyzed for 72 hours, and the product recovered by evaporation.

| | | | | |
|---|---|---|---|---|
| Analysis: | Calculated for $C_{16}H_{16}F_{17}NO_3S$; | C 30.8; | H 2.4; | N 2.2. |
| | Found | C 30.0; | H 2.3; | N 1.8. |

Example 2

$C_6F_{13}CH_2CH_2SCH_2CHOHCH_2{}^{\oplus}NHCH(CO_2{}^{\ominus})CH_2CO_2Na$

A 50 ml flask was charged with 3-(1,1,2,2-tetrahydroperfluorooctanethio)-1,2-epoxypropane (3.0 g; 6.9 mmoles), aspartic acid (1.0 g, 7.2 mmoles), 50% aqueous sodium hydroxide (0.6 g, 7.5 mmoles), isopropanol (12.6 g) and sufficient water (19.2 g) to attain a clear solution. The solution was heated to 70°C for 24 hours. After cooling, the solvent was removed by evaporation and the recovered solids were redissolved in water. The insolubles were filtered off, the filtrate dialyzed for 72 hours, and the product recovered by evaporation.

| | | | | |
|---|---|---|---|---|
| Analysis: | Calculated for $C_{15}H_{14}F_{13}NNaO_5S$; | C 28.1; | H 2.5; | N 2.5. |
| | Found | C 29.7; | H 2.6; | N 2.0. |

The surface tension depressant effect of this compound was demonstrated in N/10 sodium hydroxide solution. When used in an amount of 0.01 and 0.001 %, the surface determined was 17.8 and 34.5 dynes/cm, respectively.

Example 3

$C_{10}F_{21}CH_2CH_2SCH_2CHOHCH_2N(CH_3)CH_2CH_2SO_3Na$

A 50 ml flask was charged with 3-(1,1,2,2-tetrahydroperfluorododecanethio)-1,2-epoxypropane (6.30 g; 10 mmoles), N-methyltaurine (6.21 g; 15 mmoles), 25% aqueous sodium hydroxide (0.10 g; 20 mmoles) and 20 g of methanol. The solution was heated to 75°C for 16 hours. After cooling, 100 ml of water and 400 ml of methanol were added, the solution filtered at 72°C, cooled to 0—10°C, refiltered and dried in vacuo at 70°C.

| | | | | | | |
|---|---|---|---|---|---|---|
| Analysis: | Calculated for $C_{18}H_{17}F_{21}NNaO_4S_2$; | C 27.1; | H 2.2; | N 1.8; | F 50.0; | S 8.0. |
| | Found | C 27.2; | H 2.2; | N 1.3; | F 52.3; | S 7.5. |

Example 4

$C_{10}F_{21}CH_2CH_2SCH_2CHOHCH_2{}^{\oplus}NH(CH_2CO_2H)CH_2CO_2{}^{\ominus}$

A 50 ml flask was charged with 3-(1,1,2,2-tetrahydroperfluorododecanethio)-1,2-epoxypropane (6.30 g; 10 mmoles), iminodiacetic acid (2.93 g; 15 mmoles), 25% aqueous sodium hydroxide (3.20 g; 20 mmoles) and 20 g of methanol. The reaction mixture was heated to 75°C for 16 hours. After cooling, it was acidified with hydrochloric acid, filtered, washed with methanol and dried in vacuo at 70°C.

| | | | |
|---|---|---|---|
| Analysis: | Calculated for $C_{19}H_{16}F_{21}NO_5S$: | C 29.7; | H 2.1. |
| | Found | C 28.7; | H 2.1. |
| | (Nitrogen analysis inconsistent). | | |

The $^1H$—NMR-spectrum was consistent with the expected structure of the above compound: δ 2.35 (2H, m) $CF_2CH_2$; δ 2.72 (4H, m) $CF_2CH_2CH_2SCH_2$; δ 3.36 (2H, S) $NHCH_2CO_2{}^{\ominus}$; δ 3.43 (2H, S) $NHCH_2CO_2H$, δ 3.49 (2H) $CHOHCH_2NH$; δ 3.27 (1H, Quintet) CHOH.

Example 5

$C_{10}F_{12}CH_2CH_2SCH_2CHOHCH_2{}^{\oplus}NH_2CH(CO_2H)CH_2CO_2{}^{\ominus}$

A 50 ml flask was charged with 3-(1,1,2,2-tetrahydroperfluorododecanethio)-1,2-epoxypropane (6.30 g; 10 mmoles), aspartic acid (2.0 g; 15 mmoles) 25% aqueous sodium hydroxide (3.2 g; 20 mmoles) and 20 g of methanol. The reaction mixture was heated to 75°C for 16 hours. After cooling, it was acidified with hydrochloric acid, filtered, cooled to 0—10°C, refiltered and dried.

| | | | | |
|---|---|---|---|---|
| Analysis: | Calculated for $C_{19}H_{16}F_{21}NO_5S$: | C 29.7; | H 2.1; | N 1.8. |
| | Found | C 28.7; | H 1.8; | N 1.1. |

The $^1H$—NMR spectrum was consistent with the expected structure of the above compound: δ 2.34 (2H, m) $CF_2CH_2$; δ 2.71 (6H, m) $CH_3$—S—$CH_2$, $CH_3CO_2H$; δ 3.53 (5H, m) $CH_2$-${}^{\oplus}N$—$CH_2$, CHOH.

**Claims:**

1. A perfluoroalkyl thioalkylene amphoteric compound of the formula

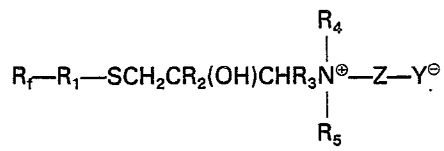

(1)

wherein

$R_f$ is perfluoroalkyl of 6 to 12 carbon atoms or said perfluoroalkyl substituted by perfluoroalkoxy of 2 to 6 atoms.

$R_1$ is alkylene of 2 to 8 carbon atoms, alkylenethioalkylene of 2 to 8 carbon atoms, alkyleneoxyalkylene of 2 to 8 carbon atoms or alkyleneiminoalkylene of 2 to 8 carbon atoms where the nitrogen atom contains hydrogen or lower alkyl as a third substituent,

$R_2$ and $R_3$ are independently hydrogen or lower alkyl which is unsubstituted or substituted by lower alkoxy or halogen, cycloalkyl of 5 or 6 carbon atoms or alkenyl of up to 6 carbon atoms or when taken together with or without a sulfur or oxygen atom, $R_2$ and $R_3$ contain up to 6 alkylene carbon atoms,

$R_4$ and $R_5$ are independently hydrogen or alkyl of 1 to 12 carbon atoms or phenyl which are unsubstituted or substituted by carboxy, sulfo, sulfato, phosphoro or phosphono,

Z is alkylene of 1 to 12 carbon atoms and

Y is carboxy, sulfo, sulfato, phosphoro, phosphono or thiosulfato,

or the acid adduct, alkaline salt, or N-lower alkyl or N-benzyl quaternary ammonium salts of the compounds of the formula (1).

2. A compound according to claim 1, wherein $R_f$ is perfluoroalkyl of 6 to 12 carbon atoms or said perfluoroalkyl substituted by perfluoroalkyl of 2 to 6 atoms,

$R_1$ is alkylene of 2 to 8 carbon atoms, alkylenethioalkylene of 2 to 8 carbon atoms, alkyleneoxyalkylene of 2 to 8 carbon atoms or alkyleneiminoalkylene of 2 to 8 carbon atoms where the nitrogen atom contains hydrogen or alkyl of 1 to 6 carbon atoms as a third substituent,

$R_2$ and $R_3$ are hydrogen;

$R_4$ and $R_5$ are hydrogen, methyl or alkyl substituted by carboxy, and

Z is alkylene of 1 to 3 carbon atoms and

Y is carboxy or sulfo,

or the acid adduct, alkaline salt or N-lower alkyl quaternary ammonium salt of the compounds of the formula (1).

3. A compound according to claim 2, wherein $R_f$ is perfluoroalkyl of 6 to 12 carbon atoms, $R_1$ is alkylene of 2 to 4 carbon atoms, $R_2$ and $R_3$ are hydrogen, $R_4$ and $R_5$ are hydrogen, methyl or lower alkyl substituted by carboxy and Z is alkylene of 1 to 3 carbon atoms and

Y is carboxy or sulfo,

or the acid adduct, alkaline salt or N-lower alkyl quaternary ammonium salt of the compounds of the formula (1).

4. A process for the preparation of the compounds of the formula (1) which comprises reacting the compound of the formula

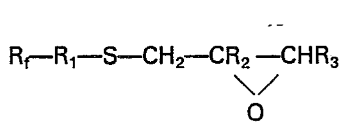

(2)

with the compound of the formula

$$NR_4R_5ZY \qquad (3)$$

wherein $R_f$, $R_1$, $R_2$, $R_3$, $R_4$, $R_5$, Z and Y are defined in claim 1, optionally in the presence of a base, in an aqeuous solvent mixture.

5. Use of the compounds of the formula (1) as surfactants and wetting agents.

**Patentansprüche:**

1. Amphotere Perfluoralkylthioalkylen-Verbindungen der Formel (1)

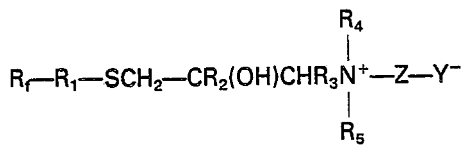

(1)

worin

$R_f$ Perfluoralkyl mit 6 bis 22 C-Atomen oder besagtes durch Perfluoralkoxy mit 2 bis 6 C-Atomen substituiertes Perfluoralkyl ist,

$R_1$ Alkylen, Alkylenthioalkylen, Alkylenoxyalkylen oder Alkyleniminoalkylen je mit 2 bis 8 C-Atomen ist, wobei das N-Atom im Alkyleniminoalkylen Wasserstoff oder Niederalkyl als dritten Substituenten enthält,

$R_2$ und $R_3$ unabhängig voneinander Wasserstoff oder unsubstituiertes oder durch Niederalkoxy oder Halogen substituiertes Alkyl, $C_5$- oder $C_6$-Cycloalkyl oder Alkenyl mit bis zu 6 C-Atomen, oder zusammen Alkylen mit bis zu 6 C-Atomen, das durch Schwefel oder Sauerstoff unterbrochen sein kann, sind,

$R_4$ und $R_5$ unabhängig voneinander Wasserstoff oder unsubstituiertes oder durch Carboxy, Sulfo, Sulfato, Phosphoro oder Phosphono substituiertes $C_1$—$C_{12}$-Alkyl oder Phenyl sind,

Z $C_1$—$C_{12}$-Alkylen und

Y Carboxy, Sulfo, Sulfato, Phosphoro, Phosphono oder Thiosulfato ist, ihre Säureaddukte, Alkalisalze oder quaternären Ammoniumsalze mit N-Niederalkyl- oder N-Benzylgruppen.

2. Verbindungen gemäss Anspruch 1, worin $R_f$, $C_6$—$C_{12}$-Perfluoralkyl oder besagtes durch $C_2$—$C_6$-Perfluoralkoxy substituiertes Perfluoralkyl,

$R_1$ Alkylen, Alkylenthioalkylen, Alkylenoxyalkylen oder Alkyleniminoalkylen je mit 2 bis 8 C-Atomen ist, wobei das N-Atom im Alkyleniminoalkylen Wasserstoff oder $C_1$—$C_6$-Alkyl als dritten Substituenten enthält,

$R_2$ und $R_3$ Wasserstoff sind,

$R_4$ und $R_5$ Wasserstoff, Methyl oder durch Carboxy substituiertes Alkyl sind,

Z $C_1$—$C_3$-Alkylen und

Y Carboxy oder Sulfo sind,

ihre Säureaddukte, Alkalisalze oder N-Niederalkyl-quaternären Ammoniumsalze.

3. Verbindungen gemäss Anspruch 2, worin

$R_f$ $C_6$—$C_{12}$-Perfluoralkyl, $R_1$ $C_2$—$C_4$-Alkylen, $R_2$ und $R_3$ Wasserstoff,

$R_4$ und $R_5$ Wasserstoff, Methyl oder durch Carboxy substituiertes Niederalkyl sind, Z $C_1$—$C_3$-Alkylen ist, und Y Carboxy oder Sulfo bedeutet,

ihre Säureaddukte, Alkalisalze oder N-Niederalkyl-quaternären Ammoniumsalze.

4. Verfahren zur Herstellung von Verbindungen der Formel (1) gemäss Anspruch 1, dadurch gekennzeichnet, dass man eine Verbindung der Formel (2)

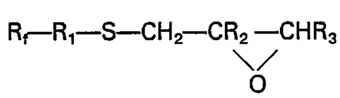

(2)

mit einer Verbindung der Formel (3)

$$NR_4R_5ZY \quad (3)$$

worin $R_f$, $R_1$, $R_2$, $R_3$, $R_4$, $R_5$, Z und Y die in Formel (1) angegebene Bedeutung haben, gegebenenfalls in Gegenwart einer Base, in einem wässrigen Reaktionsmedium umsetzt.

5. Verwendung von Verbindungen der Formel (1) gemäss Anspruch 1 als oberflächenaktive Verbindungen und als Netzmittel.

**Revendications:**

1. Composés amphotères renfermant un radical perfluoroalkyl-thio-alkylène et répondant à la formule 1:

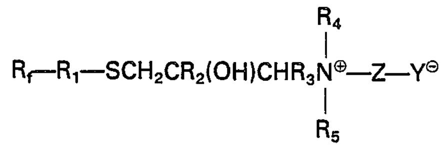
(1)

dans laquelle:

$R_f$ représente un radical perfluoroalkyle contenant de 6 à 12 atomes de carbone ou un radical perfluoroalkyle de ce genre qui porte un radical perfluoroalcoxy contenant de 2 à 6 atomes de carbone,

$R_1$ représente un radical alkylène contenant de 2 à 8 atomes de carbone, un radical alkylène-thio-alkylène contenant de 2 à 8 atomes de carbone, un radical alkylène-oxy-alkylène contenant de 2 à 8 atomes de carbone ou un radical alkylène-imino-alkylène contenant de 2 à 8 atomes de carbone dans lequel l'atome d'azote porte de l'hydrogène ou un alkyle inférieur comme troisième substituant,

$R_2$ et $R_3$ représentent chacun, indépendamment l'un de l'autre, l'hydrogène, un alkyle inférieur non substitué ou porteur d'un alcoxy inférieur ou d'un halogène, un cycloalkyle à 5 ou 6 atomes de carbone, ou un alcényle à au plus 6 atomes de carbone, ou encore $R_2$ et $R_3$ représentent ensemble, avec ou sans atome de soufre ou d'oxygène, un radical alkylène pouvant contenir jusqu'à 6 atomes de carbone,

$R_4$ et $R_5$ représentent chacun, indépendamment l'un de l'autre, l'hydrogène, un alkyle contenant de 1 à 12 atomes de carbone ou un phényle, ce radical alkyle et ce radical phényle étant dépourvus de substituant ou portant des radicaux carboxy, sulfo, sulfato, phosphato ou phosphono,

Z représente un alkylène contenant de 1 à 12 atomes de carbone et

Y représent un radical carboxy, sulfo, sulfato, phosphato, phosphono ou thio-sulfato,

ainsi que les dérivés des composés de formule 1 qui sont des produits d'addition d'acides, des sels de métaux alcalins ou des sels de N-alkyl-ammoniums quaternaires à alkyles inférieurs ou de N-benzyl-ammoniums quaternaires.

2. Composés selon la revendication 1 dans lesquels:

$R_f$ représente un radical perfluoroalkyle contenant de 6 à 12 atomes de carbone ou un radical perfluoroalkyle de ce genre qui porte un radical perfluoroalcoxy contenant de 2 à 6 atomes,

$R_1$ représente un radical alkylène contenant de 2 à 8 atomes de carbone, un alkylène-thio-alkylène contenant de 2 à 8 atomes de carbone, un alkylène-oxy-alkylène contenant de 2 à 8 atomes de carbone ou un alkylène-imino-alkylène qui contient de 2 à 8 atomes de carbone et dans lequel l'atome d'azote porte un atome d'hydrogène ou un radical alkyle contenant de 1 à 6 atomes de carbone comme troisième substituant,

$R_2$ et $R_3$ représentent chacun l'hydrogène,

$R_4$ et $R_5$ représentent chacun l'hydrogène, un méthyle ou un alkyle qui porte un radical carboxy,

Z représente un alkylène contenant de 1 à 3 atomes de carbone et

Y représente un radical carboxy ou sulfo,

et les dérivés de ces composés de formule 1 qui sont produits d'addition d'acides, des sels de métaux alcalins ou des sels de N-alkyl-ammoniums quaternaires à alkyles inférieurs.

3. Composés selon la revendication 2 dans lesquels:

$R_f$ représente un radical perfluoroalkyle contenant de 6 à 12 atomes de carbone,

$R_1$ représente un radical alkylène contenant de 2 à 4 atomes de carbone,

$R_2$ et $R_3$ représentent chacun l'hydrogène,

$R_4$ et $R_5$ représentent chacun l'hydrogène, un méthyle ou un alkyle inférieur porteur d'un carboxy,

Z représente un alkylène contenant de 1 à 3 atomes de carbone et

Y représente un carboxy ou un sulfo,

ainsi que les produits d'addition d'acides, les sels de métaux alcalins et les sels d'alkyl-ammoniums quaternaires à alkyles inférieurs de ces composés de formule 1.

4. Procédé de préparation de composés répondant à la formule 1, procédé selon lequel on fait réagir un composé de formule 2:

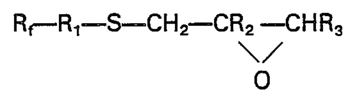
(2)

avec un composé de formule 3:

$$NR_4B \quad (3)$$

formules dans lesquelles $R_f$, $R_1$, $R_2$, $R_3$, $R_4$, $R_5$, . ont les significations données à la revendication 1, éventuellement en présence d'une base, dans ,mélange solvant aqueux.

5. Application des composés de formule 1 comme surfactifs et mouillants.